## Europäisches Patentamt
## European Patent Office
## Office européen des brevets

(19)

(11) Veröffentlichungsnummer: **0 122 515**
**B1**

(12) ## EUROPÄISCHE PATENTSCHRIFT

(45) Veröffentlichungstag der Patentschrift:
27.04.88

(21) Anmeldenummer: **84103296.4**

(22) Anmeldetag: **26.03.84**

(51) Int. Cl.⁴: **C 07 C 69/593**, C 07 C 69/60,
C 09 D 5/02, C 09 D 3/727

(54) Ungesättigte Ester, Verfahren zu ihrer Herstellung und ihre Verwendung zur Herstellung von Überzugsmitteln.

(30) Priorität: **02.04.83 DE 3312168**

(43) Veröffentlichungstag der Anmeldung:
**24.10.84 Patentblatt 84/43**

(45) Bekanntmachung des Hinweises auf die Patenterteilung:
**27.04.88 Patentblatt 88/17**

(84) Benannte Vertragsstaaten:
**AT BE DE FR GB IT NL**

(56) Entgegenhaltungen:
**Keine Entgegenhaltungen**

(73) Patentinhaber: **BAYER AG, Konzernverwaltung RP Patentabteilung, D-5090 Leverkusen 1 Bayerwerk (DE)**

(72) Erfinder: **Meixner, Jürgen, Dr., Bethelstrasse 18, D-4150 Krefeld 1 (DE)**
Erfinder: **Traenckner, Hans-Joachim, Dr., Amselweg 2, D-3032 Fallingbostel (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 122 515 B1

**0 122 515**

## Beschreibung

Die Erfindung betrifft Butendiolreste enthaltende monomere, oligomere und polymere Ester, ein Verfahren zu ihrer Herstellung und ihre Verwendung zur Herstellung von - vorzugsweise wäßrigen - Überzugsmitteln.

Die gebräuchlichsten Polyester-Überzugsmittel enthalten $\alpha, \beta$-ethylenisch ungesättigte Polyester und damit copolymerisierbare Monomere. Als Monomer wird fast ausschließlich Styrol eingesetzt. Während der Verarbeitung verdunstet Styrol, was die kostspielige Reinigung der Luft der Fabrikationshallen und der Abluft der Trockenanlagen notwendig macht.

Ungesättigte Polyester, die frei von copolymerisierbaren Monomeren sind und durch UV-Bestrahlung gehärtet werden können, werden z. B. in der DE-PS-2 221 335 beschrieben. in Anwesenheit von Peroxiden härtbare monomerfreie wäßrige Emulsionen auf Basis ungesättigter Polyester sind aus der DE-OS-2 804 216 bekannt. Beide Systeme enthalten ungesättigte Polyester mit Resten von Allylethern mehrwertiger Alkohole.

Bei der industriellen Lackierung von Holz und holzähnlichen Werkstoffen haben sich die wäßrigen Emulsionen der DE-OS-2 804 216 sehr bewährt. Verwendet man die gleichen Polyester jedoch als Grundlage für Malerlacke, so treten infolge einer Nebenreaktion in geringer Menge Abspaltprodukte auf, die zu einer Geruchsbelästigung führen.

Aufgabe der Erfindung war es nun, ungesättigte Ester herzustellen, die sich zur Herstellung monomerfreier wäßriger Emulsionen eignen und in Gegenwart von Beschleunigern mit oder ohne Peroxid ohne die oben geschilderten Nachteile gehärtet werden können.

Überraschenderweise wurde nun gefunden, daß monomere, oligomere und polymere Ester, die keine Allylethergruppen, aber die Reste von a) oxalkyliertem Buten-2-diol-1.4 und b) Buten-1-diol-3.4 und/oder oxalkyliertem Buten-1-diol-3.4 enthalten, die gestellten Anforderungen erfüllen. Sie können sikkativiert sowohl mit als auch ohne Hydroperoxid gehärtet werden und insbesondere in Form wäßriger Emulsionen zur Herstellung dünnschichtiger Überzüge dienen.

Gegenstand der Erfindung sind ungesättigte Ester, deren Säurekomponente A zu 50 - 100 Mol-%, bezogen auf A, aus $\alpha,\beta$-ethylenisch ungesättigten Dicarbonsäureresten mit 4 oder 5 C-Atomen und

50 - 0 Mol-%, bezogen auf A, aus Resten aliphatischer gesättigter Dicarbonsäuren mit 4 - 10 C-Atomen, Resten cycloaliphatischer Dicarbonsäuren mit 8 - 10 C-Atomen und/oder Resten aromatischer Dicarbonsäuren mit 8 - 12 C-Atomen besteht, dadurch gekennzeichnet, daß ihre Alkoholkomponente B pro Mol Dicarbonsäurereste A aus

a) 0,1 - 1, vorzugsweise 0,3 - 1, Mol oxalkylierten Buten-2-diol-1.4-resten,

b) 0,1 - 1, vorzugsweise 0,3 - 1, Mol Buten-1-diol-3.4-resten und/oder oxalkylierten Buten-1-diol-3.4-resten und

c) 0 - 0,8, vorzugsweise 0 - 0,4, Mol anderer Alkoholreste besteht mit der Maßgabe, daß die Summe a) + b) + c) 2 Mol nicht übersteigt.

Bei den $\alpha,\beta$-ethylenisch ungesättigen Dicarbonsäureresten handelt es sich um Reste von $C_4$- und $C_5$-Dicarbonsäuren, wie z. B. Maleinsäure-, Fumarsäure- und Itaconsäurereste.

Bevorzugte Reste aliphatischer gesättigter $C_4$-$C_{10}$-Dicarbonsäuren sind z. B. Bernsteinsäure-, Adipinsäure- und Sebacinsäurereste.

Bevorzugte Reste cycloaliphatisoher $C_8$-$C_{10}$-Dicarbonsäuren sind z. B. Hexahydrophthalsäure-, Methylhexahydrophthalsäure-, Terrahydrophthalsäure, Methyltetrahydrophthalsäure- und Endomethylentetrahydrophthalsäurereste.

Bevorzugte Reste aromatischer $C_8$-$C_{12}$-Dicarbonsäuren sind z. B. Phthalsäure-, Iso- und Terephthalsäurereste.

Der Ausdruck "oxalkyliert" im Sinne der Erfindung bedeutet: ein- bis vierfach ethoxyliert oder propoxyliert. Da bei der Umsetzung mit Alkylenoxiden bekanntlich Gemische mit unterschiedlichem Additionsgrad entstehen, bezieht sich "ein- bis vierfach" auf den durchschnittlichen Additionsgrad pro Butendioleinheit. Dementsprechend bedeutet "zweifach ethoxyliert", daß an beide Hydroxylgruppen des Butendiols im Durchschnitt jeweils 1 Molekül Ethylenoxid addiert worden ist.

"Andere Alkoholreste" c) sind vorzugsweise die Reste zweiwertiger $C_2$-$C_{10}$-Alkohole und die Reste einwertiger $C_1$-$C_{12}$-Alkohole, wie z. B. Ethylenglykol-, Diethylenglykol-, Propandiol-1.2-, Benzylalkohol- und Isooctanolreste.

Die Säurezahlen der erfindungsgemäßen Ester sollen 0 - 50, vorzugsweise 5 - 40, die OH-Zahlen 10 - 150, vorzugsweise 10 - 130 und die als Zahlenmittel bestimmten Molekulargewichte 300 - 5.000, vorzugsweise 500 - 2.000 betragen (dampfdruckosmometrisch in Dioxan und Aceton bestimmt, wobei bei differierenden Werten der niedrigere als korrekt angesehen wird).

Die Herstellung der erfindungsgemäßen Ester kann nach bekannten Methoden, z. B. durch Schmelz- oder Azeotropveresterung der Alkohole und Säuren oder deren veresterungsfähigen Derivate erfolgen, vgl. "Methoden der Organischen Chemie" (Houben - Weyl), 4. Aufl. Bd. 14/2, Georg Thieme Verlag, Stuttgart 1961, S. 1 - 5, 21 - 33, 40 - 44.

Weiterer Gegenstand der Erfindung ist also ein Verfahren zur Herstellung der oben definierten Ester durch Veresterung der Ausgangskomponenten unter Inertgasatmosphäre bei Temperaturen von 140 - 260° C.

Um die erfindungsgemäßen Ester vor unerwünschter vorzeitiger Vernetzung zu bewahren, empfiehlt es sich, bereits bei der Herstellung der Ester 0,001 - 0,1 Gew-% Polymerisationsinhibitoren oder Antioxidantien zuzusetzen. Geeignete Stabilisatoren sind in "Methoden der Organischen Chemie" (Houben Weyl), 4. Auflage,

2

Band 14/1, S. 433 ff, Georg Thieme Verlag, Stuttgart, 1961, beschrieben, Sehr gut geeignet ist z. B. p-Benzochinon in einer Konzentration von 0,01 - 0,05 Gew.-%, bezogen auf erfindungsgemäße Ester.

Die erfindungsgemäßen Ester können aufgrund ihrer niedrigen Viskosität ohne copolymerisierbare Monomere und ohne Lösungsmittel appliziert werden. Jedoch ist es auch möglich, bis 100 Gew.-%, vorzugsweise bis 80 Gew.-%, bezogen auf erfindungsgemäße Ester, copolymerisierbare Monomere oder organische Lösungsmittel zuzusetzen.

Eine bevorzugte Applikationsform der erfindungsgemäßen Ester ist die wäßrige Emulsion.

Hierzu können die erfindungsgemäßen Ester mit Hilfe externer Emulgatoren und ggf. üblicher in der Emulsionstechnik angewandter Hilfsmittel in Wasser emulgiert werden. Als Emulgatoren kommen anionische, nichtionische, kationische, ampholytische oder hochmolekulare Stoffe sowie deren Mischungen in Frage.

Solche Emulgatoren sind z. B. in Ullmanns Encyclopädie der technischen Chemie, 4. Auflage, Bd. 10, Verlag Chemie, Weinheim 1975, S. 449 - 473, beschrieben. Bevorzugte Emulgatoren sind Polyester, die frei von oxalkylierten Buten-2-diol-1.4-resten, Buten-1-diol-3.4-resten und oxalkylierten Buten-1-diol-3.4-resten sind und zu 25 - 90 Gew.-%, bezogen auf Emulgator-Polyester, aus einkondensierten Polyalkylenglykolresten eines Molekulargewichts von 250 - 2.000 bestehen und pro 100 g Emulgator-Polyester 0,02 - 0,2 Mol gesättigte aliphatische Endgruppen, die Reste von Monocarbonsäuren und/oder einwertigen Alkoholen sind, enthalten. "Gesättigt" bedeutet in diesem Zusammenhang: "frei von copolymerisierbaren Doppelbindungen", schließt also z. B. Benzylreste nicht aus. Derartige Emulgator-Polyester sind z. B. in der DE-OS-2 905 666 beschrieben.

Die wäßrigen Emulsionen enthalten in der Regel 10 - 70, vorzugsweise 30 - 70, Gew.-%, bezogen auf Emulsion, der erfindungsgemäßen ungesättigten Ester. Sie können durch einfaches Einrühren von Wasser in das Ester/Emulgator-Gemisch oder mit Hilfe eines Dissolvers hergestellt werden. Um eine feinteilige Emulsion zu erhalten, empfiehlt es sich, das Wasser bei Temperaturen unter 30°C portionsweise zuzusetzen.

Weiterer Gegenstand der Erfindung ist also die Verwendung der oben beschriebenen ungesättigten Ester zur Herstellung von - vorzugsweise wäßrigen - Überzugsmitteln.

Zum Schutz von lichtempfindlichen Untergründen, z. B. hellen Hölzern können den Überzugsmitteln geringe Mengen UV-Absorber zugesetzt werden, z. B. 2-Hydroxy-4-methoxy-benzophenon oder die üblicherweise verwendeten Zimtsäure- und Benzotriazinderivate.

Übliche Zuschlags- und Farbstoffe, Thixotropier-, Konservierungs-, Mattierungs-, Glätt- und Verlaufsmittel sowie Pigmente und Füllstoffe, können zur Erzielung spezieller Effekte ebenfalls zugesetzt werden, sofern sie die Emulsionsstabilität und die Vernetzung nicht wesentlich beeinflussen.

Das Auftragen der Überzugsmittel auf geeignete Substrate kann mittels in der Lackindustrie üblichen Methoden, wie Sprühen, Walzen, Rakeln, Drucken, Tauchen, Fluten, Streichen erfolgen.

Geeignete Substrate sind Folien, Leder, Kunststoffe, Textilien, keramische Materialien, Metalle, vorzugsweise Holz, Papier und Kartonagen.

Unter Sikkativen werden Cobalt- und Mangansalze von Säuren wie Leinölfettsäuren, Tallölfettsäuren, Sojafettsäuren, von Harzsäuren wie Abietinsäure- und Naphthensäuren, von Essigsäure oder Isooctansäure verstanden. Bevorzugt werden Cobaltoctoat, Cobaltnaphthenat und Cobaltacetat.

Bevorzugte Hydroperoxide sind tert.-Butylhydroperoxid, Pinanhydroperoxid, Cumolhydroperoxid, 2,5-Dimethylhexan-2,5-di-hydroperoxid, Cyclohexanonhydroperoxid, Methylethylketonhydroperoxid, 1-Oxy-1'-hydroperoxy-dicyclohexylperoxid, Diisopropylbenzolmonohydroperoxid und Wasserstoffperoxid.

Die Viskositätsmessungen der nachfolgenden Beispiele wurden im Höppler-Kugelfallviskosimeter (DIN 53 015) bei 20°C durchgeführt.

Die Prozentangaben beziehen sich auf das Gewicht.

**Beispiele**

Die in der nachfolgenden Tabelle aufgeführten Ausgangskomponenten wurden bei 160 - 180°C unter einem Stickstoffstrom der Schmelzkondensation unterworfen, bis die angegebene Säurezahl erreicht war. Die erhaltenen Polyester wurden mit 2 % Kobaltoctoatlösung (2,2 % Co) und 5 % einer 50-%-igen Cyclohexanonhydroperoxid-Paste gemischt, ein Überzug von 90 μm Dicke auf Glasplatten hergestellt und bei 24°C gehärtet. Die Zeit bis zur Klebfreiheit kann der Tabelle 1 entnommen werden.

**Tabelle 1**

| Ausgangsmaterialien (g) Eigenschaften | erfindungsgemäße Beispiele | | | | | Vergleichsbeispiele | |
|---|---|---|---|---|---|---|---|
| | 1 | 2 | 3 | 4 | 5 | 6 | 7 |
| Fumarsäure | 116 | 116 | 116 | - | 116 | 116 | 116 |
| Maleinsäureanhydrid | - | - | - | 98 | - | - | - |
| Propandiol-1.2 | - | - | - | 45,7 | - | - | - |
| Buten-1-diol-3.4 | - | - | - | - | 44 | - | - |
| Buten-1-diol-3.4, 2-fach ethoxyliert | - | - | 105,6 | - | - | - | - |
| Buten-1-diol-3.4, 3-fach ethoxyliert | 110 | 110 | - | 88 | - | - | 187 |
| Buten-2-diol-1.4, 2-fach ethoxyliert | 70,4 | - | 70,4 | 52,8 | 88 | 149,6 | - |
| Buten-2-diol-1.4, 4-fach propoxyliert | - | 160 | - | - | - | - | - |
| Isooctanol | 23,4 | - | - | - | - | - | - |
| Benzylalkohol | 40 | 21,6 | 21,6 | - | 37,8 | 54 | 54 |
| Säurezahl | 11 | 23 | 32 | 34 | 43 | 15 | 18 |
| OH-zahl | 50 | 60 | 60 | 120 | 105 | 30 | 30 |
| Viskosität (mPa.s) | 18300 | 15600 | 31260 | 25000 | 17300 | 16300 | 16500 |
| klebfrei nach (Stunden) | 5 - 6 | 5 - 6 | 4 - 5 | 5 - 6 | 4 - 5 | 3 - 4 jedoch nicht kratzfest) | 10-22 |

Als Emulgator für die nachstehend beschriebenen Emulsionen wurde folgender Polyester eingesetzt:

Emulgatorpolyester

175 g Maleinsäureanhydrid, 643 g Polyethylenglykol (Molekulargewicht 400) und 214 g Trimethylolpropandibenzylether wurden unter $N_2$-Strom bei 190°C der Schmelzkondensation unterzogen. Säurezahl 7, OH-Zahl 36; Viskosität 20.500 mPa.s.

Je 200 g der ungesättigten Polyester der Beispiele 1 - 3 und der Vergleichsbeispiele 6 und 7 wurden mit je 50 g des obigen Emulgatorpolyesters und mit je 110 g Wasser gemischt, im Dissolver bei 8.000 U/min. 2 Minuten geschert und anschließend mit Wasser unter Rühren (1.000 U/min) auf 50 % Festkörpergehalt eingestellt. Es entstanden Öl-in-Wasser-Emulsionen.

Diese Emulsionen wurden

a) mit 2 % Kobaltacetatlösung (5 % Co) und 3 % einer 35-%-igen wässrigen $H_2O_2$-Lösung bzw.

b) mit 2 % Kobaltacetatlösung

versetzt und auf Glasplatten aufgezogen (Naßfilmdicke: 90 µm). Die Trocknungszeiten bei Raumtemperatur (24°C) bis zur Wischfestigkeit bzw. bis zur Klebfreiheit sind in der folgenden Tabelle 2 aufgeführt.

**Tabelle 2**

| Beispiel bzw. Vergleichsbeispiel | A Wischfestigkeit | B Klebfreiheit |
|---|---|---|
| 1 | 75 min | 8 Stunden |
| 2 | 80 min | 8 Stunden |
| 3 | 66 min | 8 Stunden |
| 6 | 48 min* | 7 Stunden** |
| 7 | 240 min | 10 Stunden |

\* nicht kratzfest
\*\* nicht kratzfest, Kräuselung des Films

**Patentansprüche**

1. Ungesättigte Ester, deren Säurekomponente A zu 50 - 100 Mol-%, bezogen auf A, aus α,β-ethylenisch ungesättigten Dicarbonsäureresten mit 4 oder 5 C-Atomen und

50 - 0 Mol-%, bezogen auf A, aus Resten aliphatischer gesättigter Dicarbonsäuren mit 4 - 10 C-Atomen, Resten cycloaliphatischer Dicarbonsäuren mit 8 - 10 C-Atomen und/oder Resten aromatischer Dicarbonsäuren mit 8 - 12 C-Atomen besteht, dadurch gekennzeichnet, daß ihre Alkoholkomponente B pro Mol Dicarbonsäurereste A aus

a) 0,1 - 1 Mol oxalkylierten Buten-2-diol-1.4-resten,

b) 0,1 - 1 Mol Buten-1-diol-3.4-resten und/oder oxalkylierten Buten-1-diol-3.4-resten und

c) 0 - 0,8 Mol anderer Alkoholreste besteht mit der Maßgabe, daß die Summe a) + b) + c) 2 Mol nicht übersteigt.

2. Ester nach Anspruch 1, dadurch gekennzeichnet, daß ihre Alkoholkomponente B pro Mol Dicarbonsäurereste A aus

0,3 - 1 Mol Resten a),
0,3 - 1 Mol Resten b) und
0 - 0,4 Mol Resten c) besteht.

3. Ester nach Ansprüchen 1 und 2, dadurch gekennzeichnet, daß sie als Zahlenmittel bestimmte Molekulargewichte von 300 - 5.000 besitzen.

4. Ester nach Ansprüchen 1 und 2, dadurch gekennzeichnet, daß sie als Zahlenmittel bestimmte Molekulargewichte von 500 - 2.000 besitzen.

5. Verfahren zur Herstellung der Ester nach Ansprüchen 1 bis 4 durch Veresterung der Ausgangskomponenten unter Inertgasatmosphäre bei Temperaturen von 140 - 260°C.

6. Verwendung der Ester nach Ansprüchen 1 - 4 zur Herstellung von - vorzugsweise wäßrigen - Überzugsmitteln.

## Claims

1. Unsaturated esters whose acid component A is composed of from 50 to 100 mol %, based on A, of $\alpha,\beta$-ethylenically unsaturated dicarboxylic acid residues containing 4 or 5 carbon atoms and

from 50 to 0 mol %, based on A, of residues of aliphatic, saturated dicarboxylic acids containing from 4 to 10 carbon atoms, residues of cycloaliphatic dicarboxylic acids containing from 8 to 10 carbon atoms and/or residues of aromatic dicarboxylic acids containing from 8 to 12 carbon atoms, characterised in that their alcohol component B is composed, per mol of dicarboxylic acid residues A, of

a) from 0.1 to 1 mol of alkoxylated butene-2-diol-(1,4) residues,

b) from 0.1 to 1 mol of butene-1-diol-(3,4) residues and/or alkoxylated butene-1-diol-(3,4) residues and

c) from 0 to 0.8 mol of other alcohol residues, with the proviso that the sum of a) + b) + c) does not exceed 2 mol.

2. Esters according to claim 1, characterised in that their alcohol component B is composed, per mol of dicarboxylic acid residues A, of

from 0.3 to 1 mol of residues a),

from 0.3 to 1 mol of residues b) and

from 0 to 0.4 mol of residues c).

3. Esters according to claims 1 and 2, characterised in that they have number average molecular weights of from 300 to 5,000.

4. Esters according to claims 1 and 2, characterised in that they have number average molecular weights of from 500 to 2,000.

5. Process for the preparation of esters according to claims 1 to 4 by the esterification of the starting components in an inert gas atmosphere at temperatures of from 140 to 260°C.

6. Use of the esters according to claims 1 to 4 for the preparation of coating compounds, preferably aqueous coating compounds.

## Revendications

1. Esters non saturés dont le composant acide A est constitué en proportion de 50 à 100 moles %, par rapport à A, de restes d'acides dicarboxyliques à non-saturation $\alpha,\beta$-éthylénique ayant 4 ou 5 atomes de carbone et

de 50 à 0 moles %, par rapport à A, de restes d'acides dicarboxyliques aliphatiques saturés ayant 4 à 10 atomes de carbone, de restes d'acides dicarboxyliques cycloaliphatiques de 8 à 10 atomes de carbone et/ou de restes d'acides dicarboxyliques aromatiques de 8 à 12 atomes de carbone, caractérisés en ce que leur composant alcool B est constitué, par mole, de restes d'acides dicarboxyliques A,

a) de 0,1 à 1 mole de restes butène-2-diol-1,4 oxalkylé,

b) de 0,1 à 1 mole de restes butène-1-diol-3,4 et/ou de restes butène-1-diol-3,4 oxalkylé et

c) de 0 - 0,8 mole d'autres restes alcool, sous réserve que la somme a) + b) + c) ne dépasse pas 2 moles.

2. Esters suivant la revendication 1, caracterisés en ce que leur composant alcool B est constitué, par mole, de restes d'acides dicarboxyliques A,

de 0,3 à 1 mole de restes a),

de 0,3 à 1 mole de restes b) et

de 0 à 0,4 mole de restes c).

3. Esters suivant les revendications 1 et 2, caractérisés en ce qu'ils possèdent des poids moléculaires de 300 à 5000, déterminés d'après la moyenne en nombre.

4. Esters suivant les revendications 1 et 2, caractérisés en ce qu'ils ont des poids moléculaires de 500 à 2000, déterminés par la moyenne en nombre.

5. Procédé de production des esters suivant les revendications 1 à 4 par estérification des composants de départ en atmosphère de gaz inerte, à des températures de 140 à 260°C.

6. Utilisation des esters suivant les revendications 1 à 4 pour la production de compositions de revêtement - de préférence aqueuses.